# NOUVEAU FASCICULE DE BREVET EUROPEEN

(11) **EP 1 406 579 B2**
(45) Date de publication et mention de la décision concernant l'opposition: **23.11.2016**
(45) Mention de la délivrance du brevet: 03.12.2008
(21) Numéro de dépôt: 02787146.6
(22) Date de dépôt: 09.07.2002
(51) Int. Cl.: A61K 8/34, A61Q 11/00

(54) **PROCEDE DE PREPARATION DE PATE DENTIFRICE METTANT EN OEUVRE UN SIROP DE SORBITOL PARTICULIER, ET SIROP DE SORBITOL**
VERFAHREN ZUR HERSTELLUNG EINER SORBITOL ENTHALTENDE ZAHNPASTE UND SORBITOLSIRUP
METHOD FOR PREPARING TOOTHPASTE USING A PARTICULAR SORBITOL SYRUP, AND SORBITOL SYRUP

(30) Priorité: 18.07.2001 FR 0109609
(43) Date de publication de la demande: 14.04.2004
(73) Titulaire: Roquette Frères, 62136 Lestrem (FR)
(72) Inventeur: FRANCOIS, Alain, F-62350 Calonne Sur La Lys (FR)
(74) Mandataire: Cabinet Plasseraud
(86) Numéro de dépôt international: PCT/FR2002/002402
(87) Numéro de publication internationale: WO 2003/007902

(56) Documents cités:
- EP-A- 0 711 743
- EP-A- 1 004 354
- EP-A- 1 095 925
- US-A- 4 357 314

## Description

L'invention se rapporte à un nouveau procédé de préparation de pâte dentifrice.

Plus précisément, l'invention a pour objet un procédé de préparation de pâte dentifrice comprenant un sirop de sorbitol particulier comme principal humectant.

Les pâtes dentifrice sont des produits bien connus de l'état de l'art. Ils comprennent généralement un abrasif et un gélifiant dans un milieu liquide consistant en un humectant et divers ingrédients tels que des arômes, des colorants, des conservateurs, des détergents, des agents anti-tartre, anti-bactériens, etc... Ces produits se présentent sous forme de pâtes, gels ou liquides, opaques, translucides ou transparents, et sont utilisés à des fins cosmétiques ou thérapeutiques.

L'humectant consiste le plus souvent en un sirop de polyol tel que le sorbitol, la glycérine, le xylitol, le mannitol et leurs mélanges. Il est utilisé pour éviter le durcissement des pâtes dentifrice au cours du stockage et peut assurer également un rôle d'édulcorant.

Le sorbitol est largement utilisé dans cette application sous forme de sirop non cristallisable, à environ 70% sur sec de richesse en sorbitol, que l'on trouve sur le marché à 70% de matière sèche comme notamment le NEOSORB®70/70 commercialisé par la Demanderesse. De manière générale, on l'utilise dans des proportions d'environ 20 à 70% en poids de la pâte dentifrice.

De nombreuses formules comprenant du sorbitol à 70% de matière sèche ont été décrites notamment dans les documents US-A-5.252.313, WO-A-95/22958, WO-A- 96/38123.

Un des points critiques dans la préparation d'une pâte dentifrice concerne la dispersion de l'agent gélifiant dans l'humectant. L'agent gélifiant consiste généralement en polysaccharides tels que les celluloses ou ses dérivés, les pectines, gélatines, agar-agar, les gommes végétales comme les alginates, carraghénanes, xanthanes ou encore les composés gélifiants inorganiques tels que les silices épaississantes ou les polyacrylates. Bien qu'hydrophile, l'agent gélifiant a tendance à former des grumeaux et à s'agglomérer. Certains fabricants contournent le problème en s'équipant de mélangeurs à très haut cisaillement, mais ce matériel est très coûteux. Une solution à ce problème a été proposée dans la demande de brevet EP-A-1.004.354 dont la Demanderesse est titulaire, qui décrit des compositions de polyols dont la matière sèche, comprise entre 83 et 90%, a été sélectionnée pour assurer une bonne dispersion des agents gélifiants.

Les fabricants de dentifrice sont intéressés par la dispersion facile des agents gélifiants dans ces compositions et par la possibilité de disposer de davantage d'eau libre pour pouvoir dissoudre les actifs et les autres ingrédients de la pâte dentifrice. Toutefois, ils sont freinés par la nécessité de chauffer l'ensemble des matériels de stockage, de manipulation et de production afin d'éviter les risques de cristallisation. De plus, lors de la préparation de dentifrices, la viscosité élevée de telles compositions qui résulte de l'excellente dispersion du gélifiant, peut s'avérer trop élevée pour l'équipement d'agitation en place.

Cherchant à améliorer l'état de la technique, la Demanderesse s'est alors aperçue qu'au sein d'une plage très particulière de matière sèche, spécifiquement sélectionnée, un sirop de sorbitol offrait la possibilité d'améliorer sensiblement la dispersion des abrasifs dans la pâte et de diminuer avantageusement le temps de production des dentifrices, sans présenter les inconvénients des sirops de sorbitol de l'art antérieur. On entend par abrasifs au sens de la présente invention des composés tels que par exemple les silices ou dérivés de silice, les phosphates de calcium, le carbonate de calcium.

La présente invention a donc pour objet un procédé de préparation de pâte dentifrice comprenant un sirop de sorbitol comme principal humectant, au moins un abrasif et au moins un agent gélifiant, **caractérisé en ce que** ledit sirop de sorbitol présente une matière sèche comprise entre 74 et 80%, une richesse en D-sorbitol comprise entre 72 et 92% en poids sur sec, et une teneur en sucres réducteurs inférieure à 500 ppm.

L'utilisation d'un tel sirop pour la préparation de pâtes dentifrice est nouvelle. Sa mise en oeuvre dans un procédé conforme à l'invention permet, de façon surprenante et inattendue, une dispersion des agents abrasifs nettement améliorée entraînant un gain de temps de production avantageux, et conduit à des produits stables et parfaitement homogènes. De très bons résultats en terme de gain de temps de mélange ont été observés lorsqu'on utilise par exemple des silices en tant qu'agent abrasif. Selon l'invention, la pâte dentifrice comprend donc de préférence en tant qu'agent abrasif un composé choisi dans le groupe constitué par les silices ou dérivés de silice, le carbonate de calcium, les phosphates de calcium. De manière encore plus préférentielle, ledit abrasif est une silice ou un dérivé de silice.

D'autre part, la mise en oeuvre du sirop de sorbitol conforme à l'invention permet pour une même concentration finale en sorbitol dans la pâte dentifrice de disposer d'une quantité plus importante d'eau libre pour dissoudre tous les ingrédients que l'on souhaite inclure dans la pâte. Il en résulte une meilleure homogénéité. La teneur totale en eau d'une pâte dentifrice préparée selon le procédé conforme à l'invention est comprise généralement entre 15 et 60% en poids, selon la texture de pâte désirée en final.

On utilise dans un procédé selon l'invention un sirop de sorbitol répondant à la pharmacopée européenne (édition 2001,0437), à savoir un sirop comprenant 72 à 92% sur sec de D-sorbitol. Avantageusement, le sirop de sorbitol conforme à l'invention est non cristallisable.

Dans la présente invention, ledit sirop de sorbitol présente avantageusement une teneur en sucres réducteurs inférieure ou égale à 500 ppm, de préférence inférieure à 300 ppm, ce qui permet de l'utiliser notamment dans des pâtes dentifrice contenant des composés de pH basique comme par exemple celles qui contiennent du bicarbonate de sodium, dans lesquelles ledit sirop présente une excellente stabilité c'est-à-dire une absence de coloration dans le temps. Cette teneur en sucres réducteurs peut être déterminée par toute technique connue de l'homme du métier et de sensibilité adaptée à des teneurs relativement faibles. Avantageusement, on utilisera le test S tel que décrit par la Demanderesse dans les documents EP-B1-0.711.743 ou EP-A1-1.095.925.

Pour préparer le sirop de sorbitol conforme à l'invention, on peut préparer un sirop à partir de sorbitol poudre en l'amenant à la richesse et à la matière sèche voulues, ou concentrer directement un sirop de sorbitol du commerce jusqu'à la matière sèche voulue. Cette concentration peut être effectuée à l'aide de dispositifs conventionnels d'évaporation.

On pourra avantageusement utiliser les sirops de sorbitol tels que décrits dans le brevet EP-B1-0.711.743 ou obtenus selon le procédé décrit dans la demande de brevet EP-A1-1.095.925 que l'on concentrera jusqu'à la matière sèche voulue.

Selon un autre mode de réalisation de l'invention, la pâte dentifrice peut comprendre un agent humectant additionnel tel que par exemple la glycérine, le xylitol, le propylène glycol, le polyéthylène glycol. De préférence, la pâte dentifrice comprend jusqu'à 20% en poids dudit humectant additionnel.

En ce qui concerne la préparation de la pâte dentifrice, on utilisera les équipements et formules connues de l'homme du métier. Selon un mode général de réalisation du procédé selon l'invention, on mélange d'abord l'humectant avec l'agent gélifiant. Cet agent gélifiant est généralement une cellulose ou un dérivé de cellulose comme par exemple une carboxyméthyl cellulose. On ajoute ensuite après homogénéisation les divers ingrédients tels qu'arômes, édulcorants, colorants, sels de fluor, agents anti-tartre, anti-bactériens, agents de blanchiment, détergents et conservateurs.

La présente description a en outre pour objet un sirop de sorbitol caractérisé en ce qu'il présente une richesse en D-sorbitol comprise entre 72 et 92%, une matière sèche comprise entre 74 et 80% et une teneur en sucres réducteurs inférieure à 500ppm, de préférence inférieure à 300ppm. De manière préférentielle, ledit sirop présente une teneur en sucres totaux après hydrolyse totale selon la méthode de Bertrand comprise entre 3,5 et 98%, de préférence entre 6 et 92% et plus préférentiellement encore entre 8 et 90 %. Il peut comprendre en outre de 0,01 à 95% de mono- et/ou de disaccharides hydrogénés, le complément à 100 étant constitué d'oligo et polysaccharides. De manière encore plus préférentielle, ledit sirop est non cristallisable.

La Demanderesse a en effet constaté que ces sirops, particulièrement adaptés à la bonne dispersion des abrasifs et autres ingrédients dans une pâte dentifrice, pouvaient avantageusement être concentrés à la matière sèche sélectionnée sans engendrer de coloration, d'odeurs ou de goûts indésirables dans l'application visée. Seule la sélection particulière de tels sirops conduit à des produits stables, incolores et exempts de goûts et d'odeurs désagréables, alors que la concentration de sirops de sorbitol classiques est une opération délicate à réaliser en raison des précautions à prendre notamment en termes de température.

L'invention sera mieux comprise à la lecture des exemples qui suivent, lesquels ne se veulent pas limitatifs et font seulement état de modes de réalisation avantageux du procédé conforme à l'invention.

### Exemple 1 préparation de pâtes dentifrice selon l'invention et comparaison avec l'art antérieur.

On prépare, à titre d'humectant, différents sirops de sorbitol présentant des matières sèches (MS) de 70% et 86,8% (art antérieur) et 74%, 78% (invention) par concentration d'un sirop de sorbitol (NEOSORB®70/70). On prépare à l'aide de ces sirops différentes pâtes dentifrice contenant toutes la même teneur en sorbitol.

La formule pour chaque dentifrice est la suivante (pourcentages exprimés en poids):
Silice (TIXOSIL 73) : 14%
Silice (TIXOSIL 43): 9%
Détergent (SIPON LCSV95, 30%MS) : 4,16%
Arôme menthe Silésia : 0,80%
Monofluorophosphate de sodium : 0,76%
Carboxyméthyl cellulose (BLANOSE 7MXF) : 0,7%
Colorant vert (0,5%MS) : 0,5%
Saccharinate de sodium : 0,2%
Conservateur (méthyl paraben) : 0,18%
Conservateur (propyl paraben) : 0,02%

| | |
|---|---|
| Pâte 1 : | NEOSORB 70/70 à 70%MS : 64% |
| | Eau : 5,68% |
| Pâte 2 : | NEOSORB à 74%MS : 60,54% |
| | Eau : 9,14% |
| Pâte 3 : | NEOSORB à 78%MS : 57,44% |
| | Eau : 12,24% |
| Pâte 4 : | NEOSORB à 86,8%MS : 51,61% |
| | Eau : 18,07% |

### Mode opératoire :

Dans un bécher, dissoudre les parabens dans l'eau à 92°C (solution S).
Dans un bol en inox, mélanger le sirop de sorbitol et la carboxyméthyl cellulose pendant 1 minute.
Ajouter la solution S, mélanger pendant 2 min.
Homogénéiser pendant 3 min.
Transvaser dans un homogénéiseur GUEDU (type 4.5NO) à 20°C.
Ajouter le saccharinate, le monofluorophosphate, le colorant et l'arôme, mélanger pendant 1 min 30s sous vide.
Ajouter la moitié des silices, mélanger 3 min sous vide.
Ajouter la seconde moitié des silices, mélanger 3 min sous vide.
Mélanger pendant un temps T jusqu'à homogénéisation.
Ajouter le SIPON, mélanger 1 min 30s sous vide.

On mesure pour chaque pâte préparée le temps total de fabrication, le temps T nécessaire à la dispersion des silices, la viscosité après fabrication et la viscosité après 24 heures (appareil HELIPATH, viscosité en centipoises).

Les résultats sont repris dans le tableau suivant.

| | **PÂTE 1** | **PÂTE 2** | **PÂTE 3** | **PÂTE 4** |
|---|---|---|---|---|
| Temps total | 30min30s | 28min30s | 21min30s | - |
| Temps T de mélange des silices | 21min30s | 19min30s | 12min30s | ** |
| Viscosité à To (cps) | 360000 | 360000 | 460000 | |
| Viscosité à 24h (cps) | 440000 | 460000 | 520000 | |
| ** dans ce cas, l'incorporation de la deuxième moitié des silices est incomplète car la viscosité du mélange est trop élevée pour le mélangeur utilisé. | | | | |

Observations : les sirops de sorbitol conformes à l'invention réduisent considérablement le temps nécessaire à une dispersion parfaitement homogène des silices. On constate d'après les formules selon l'invention que la quantité d'eau disponible est augmentée (jusqu'à 12% d'eau) ce qui améliore la dispersion et/ou solubilisation des autres ingrédients. Les viscosités sont meilleures, ce qui entraîne une texture de pâte améliorée et un temps de mélange des silices moins long.

Dans le cas de la pâte 4, préparée avec un sirop de sorbitol à 86,8% de matière sèche, la dispersion des silices est insuffisante.

La mise en oeuvre de sirops de sorbitol conformes à l'invention pour la préparation de pâtes dentifrice est donc techniquement et économiquement tout à fait avantageuse (gain de temps important, qualité des pâtes améliorée). De tels sirops sont de plus tout à fait avantageux en termes d'économie de coût de transport, diminution des volumes à stocker, facilité de mise en oeuvre.

### Exemple 2 formulation de pâtes dentifrice au carbonate de calcium.

On prépare des pâtes dentifrice conformément à l'exemple 1 en remplaçant les silices abrasives par du carbonate de calcium.

On prépare des sirops de sorbitol (NEOSORB®70/70) à 70, 75 et 80% de matière sèche (pâtes 1, 2 et 3). Les teneurs en sorbitol de chaque pâte sont identiques.

La formule est la suivante :
Carbonate de calcium (SOCAL 90A) _{:} 45%
Sipon LCSV95 (30%MS) : 5,66%
Blanose 7MXF _{:} 1,2%
Arôme menthe Silesia : 1%
Monofluorophosphate de sodium : 0,8%
Saccharinate de sodium : 0,2%
Methyl paraben : 0,18%
Propyl paraben : 0,02%

| | |
|---|---|
| Pâte 1 : | NEOSORB 70/70 à 70%MS : 35,7% |
| | Eau : 10,29% |
| Pâte 2 : | NEOSORB à 75%MS : 33,32% |
| | Eau : 12,67% |
| Pâte 3 : | NEOSORB à 80%MS : 31,24% |
| | Eau : 14,75% |

### Mode opératoire :

Dans un bécher, dissoudre les parabens dans l'eau à 72°C : solution S
Dans un bol en inox, mélanger le sirop de sorbitol et la carboxyméthyl cellulose pendant 1 minute.
Ajouter la solution S, mélanger pendant 2 min.
Homogénéiser pendant 3 min.
Transvaser dans le mélangeur GUEDU à 20°C.
Ajouter le saccharinate, le monofluorophosphate et l'arôme, mélanger pendant 1 min 30s sous vide.
Ajouter le carbonate, mélanger 3 min sous vide.
Ajouter le Sipon, mélanger 1 min 30s sous vide.

On mesure pour chaque pâte préparée le temps total de fabrication, le temps T nécessaire à la dispersion du carbonate.

Les résultats sont repris dans le tableau suivant.

| | **PÂTE 1** | **PÂTE 2** | **PÂTE 3** |
|---|---|---|---|
| Temps total de fabrication | 15 min | 13 min 30 s | 12 min |
| Temps T de mélange du carbonate | 6 min | 4 min 30 s | 3 min |

Ces résultats démontrent que les temps de mélange de l'abrasif sont nettement améliorés lorsqu'on met en oeuvre les sirops de sorbitol conformes à l'invention.

Le temps total de fabrication des pâtes dentifrice s'en trouve avantageusement écourté.

### Exemple 3 formulation de pâtes dentifrice au bicarbonate de sodium.

On prépare, à titre d'humectant, différents sirops de sorbitol présentant des matières sèches (MS) de 70% (art antérieur) et 74%, 78% (invention) par concentration d'un sirop de sorbitol non cristallisable présentant une teneur en sucres réducteurs inférieure à 500ppm, une teneur en sucres totaux de 7% après hydrolyse totale selon la méthode de Bertrand, et comprenant 88% de mono-et/ou de disaccharides hydrogénés, le complément à 100 étant constitué d'oligo et polysaccharides (NEOSORB® 70/70 SB).

On prépare à l'aide de ces sirops différentes pâtes dentifrice au bicarbonate de sodium contenant toutes la même teneur en sorbitol sec (31,5%).

La formule pour chaque dentifrice est la suivante (pourcentages exprimés en poids):
NEOSORB®70/70SB: 45,00%
Eau : 16,94%
Bicarbonate de sodium : 10,00%
Silice épaississante Tixosil 43 : 10,00%
Silice abrasive Tixosil 73 : 9,00%
Lauryl sulfate de sodium Sipon LCSV95(30%MS) : 5,66%
Arôme menthe : 1,00%
Monofluorophosphate de sodium : 0,8%
Carboxyméthylcellulose sodique Blanose 7MXF : 0,7%
Dioxyde de titane : 0,7%
Saccharinate de sodium : 0,2%

Le mode opératoire pour chaque pâte diffère uniquement par l'application d'un temps de mélange supplémentaire ou non. Ce temps de mélange supplémentaire a pour but l'élimination éventuelle de grains résiduels dans la pâte.

### Mode opératoire :

- dans un bécher, dissoudre le monofluorophosphate et le saccharinate dans l'eau pour obtenir une solution S ;
- dans le bol inox, mélanger le NEOSORB® et la CMC pendant 1 min. (moteur+turbine) ;
- ajouter la solution S et l'oxyde de titane, mélanger 2 min.;
- homogénéiser pendant 3 min.;
- transvaser dans un mélangeur GUEDU ;
- ajouter l'arôme et mélanger sous vide pendant 1 min. 30 sec ;
- Ajouter la silice épaississante et mélanger sous vide pendant 3 min. ;
- Ajouter la silice abrasive et mélanger sous vide pendant 3 min. ;
- Ajouter le bicarbonate et mélanger sous vide pendant 3 min ;
- Mélanger T min. supplémentaires (selon le sirop de sorbitol employé) ;
- Ajouter le Sipon et mélanger sous vide pendant 1 min. 30 sec.

On mesure pour chaque pâte préparée le temps total de fabrication, le temps T supplémentaire nécessaire à la dispersion des silices, la viscosité après fabrication et la viscosité après refroidissement (appareil HELIPATH, viscosité en centipoises).

Les résultats sont repris dans le tableau ci-après :

| | | | |
|---|---|---|---|
| Matière sèche du sirop de sorbitol (%) | 70 | 74 | 78 |
| **Temps de mélange supplémentaire (min)** | **9** | **6** | **3** |
| Temps de mélange total (min) | 27 | 24 | 21 |
| Viscosité pâte après fabrication (cP) | 230 000 | 310 000 | 230 000 |
| Viscosité pâte après refroidissement (cP) | 380 000 | 500 000 | 580 000 |

Commentaires : ces résultats démontrent, comme dans les exemples précédents, que les sirops de sorbitol conformes à l'invention permettent avantageusement un temps de mélange d'autant plus court que leur matière sèche est élevée, et permettent une meilleure dispersion des silices, ainsi qu'une parfaite hydratation de la carboxyméthylcellulose, qui se traduit par une viscosité de pâte plus élevée après fabrication.

## Revendications

1. Procédé de préparation de pâte dentifrice comprenant un sirop de sorbitol comme principal humectant, au moins un abrasif et au moins un agent gélifiant, **caractérisé en ce que** l'on met en oeuvre à titre d'humectant, un sirop de sorbitol présentant une richesse en D-sorbitol comprise entre 72 et 92% en poids sur sec, une matière sèche comprise entre 74 et 80%, et une teneur en sucres réducteurs inférieure à 500ppm, de préférence inférieure à 300ppm.

2. Procédé selon la revendication 1, **caractérisé en ce que** ledit sirop de sorbitol présente une teneur en sucres réducteurs inférieure ou égale à 300 ppm.

3. Procédé selon l'une ou l'autre des revendications 1 ou 2, **caractérisé en ce que** ledit abrasif est choisi dans le groupe constitué par les silices, dérivés de silice, carbonate de calcium ou phosphates de calcium.

4. Procédé selon l'une quelconque des revendications 1 à 3, **caractérisé en ce que** ledit abrasif est une silice ou un dérivé de silice.

5. Procédé selon l'une quelconque des revendications 1 à 4, **caractérisé en ce que** ladite pâte dentifrice comprend jusqu'à 20% en poids d'un humectant additionnel.

6. Procédé selon l'une quelconque des revendications 1 à 5, **caractérisé en ce que** ledit humectant additionnel est la glycérine.

7. Sirop de sorbitol **caractérisé en ce qu'**il présente une richesse en D-sorbitol comprise entre 72 et 92% en poids sur sec, une matière sèche comprise entre 74 et 80%, et une teneur en sucres réducteurs inférieure à 500 ppm, de préférence inférieure à 300 ppm.

## Patentansprüche

1. Verfahren zur Herstellung von Zahnpasta, enthaltend einen Sorbitolsirup als Hauptfeuchthaltemittel, mindestens ein Abrasivum und mindestens ein Gelierungsmittei, **dadurch gekennzeichnet, daß** man als Feuchthaltemittel einen Sorbitolsirup mit einem Gehalt an D-Sorbitol zwischen 72 und 92 Trockengew.-%, einen Feststoffgehalt zwischen 74 und 80% und einen Gehalt an reduzierenden Zuckern von weniger als 500 ppm und vorzugsweise weniger als 300 ppm verwendet.

2. Verfahren nach Anspruch 1, **dadurch gekennzeichnet, daß** der Sorbitolsirup einen Gehalt am reduzierenden Zuckern kleiner gleich 300 ppm, aufweist.

3. Verfahren nach einem der Ansprüche 1 oder 2, **dadurch gekennzeichnet, daß** man des Abrasivum aus der Gruppe bestehend aus Siliciumdioxiden, Siliciumioxidderivaten, Galciumcarbonat oder Calciumphosphaten auswählt.

4. Verfahren nach einem der Ansprüche 1 bis 3, **dadurch gekennzeichnet, daß** es sich bei dem Abrasivum um ein Siliciumdioxid oder ein Siliciumdioxidderivat handelt.

5. Verfahren nach einem der Ansprüche 1 bis 4, **dadurch gekennzeichnet, daß** die Zahnpasta bis zu 20 Gew.-% eines zusätzliche Feuchthaltemittels enthält.

6. Verfahren nach einem der Ansprüche 1 bis 5, **dadurch gekennzeichnet, daß** es sich bei dem zusätzlichen Feuchthaltemittel um Glycerin handelt.

7. Sorbitolsirup, **dadurch gekennzeichnet, daß** er einen Gehalt an D-Sorbitol zwischen 72 und 92 Trockengew.-%, einen Feststoffgehalt zwischen 74 und 80% und einen Gehalt an reduzierenden Zuckern von weniger als 500 ppm und vorzugsweise weniger als 300 ppm aufweist.

## Claims

1. A method for preparing toothpaste comprising a sorbitol syrup as main humectant, at least one abrasive agent and at least one gelling agent, **characterized in that** a sorbitol syrup having D-sorbitol content of between 72 and 92% by weight on a dry basis, a dry matter content of between 74 and 80%, a reducing sugar content of less than 500 ppm, preferably of less than 300 ppm, is used as humectant.

2. The method as claimed in claim 1, **characterized in that** said syrup has a reducing sugar content of less than or equal to 300 ppm.

3. The method as claimed in any one of claims 1 or 2, **characterized in that** said abrasive is selected from the group consisting of silicas, silica derivatives, calcium carbonate or calcium phosphates.

4. The method as claimed in any one of claims 1 to 3, **characterized in that** said abrasive is a silica or a silica derivative.

5. The method as claimed in any one of claims 1 to 4, **characterized in that** said toothpaste comprises up to 20% by weight of an additional humectant.

6. The method as claimed in any one of claims 1 to 5, **characterized in that** said additional humectant is glycerin.

7. A sorbitol syrup **characterized in that** it has a D-sorbitol content of between 72 and 92% by weight on a dry basis, a dry matter content of between 74 and 80%, a reducing sugar content of less than 500 ppm, preferably of less than 300 ppm.
